# FASCICULE DE BREVET EUROPEEN

(11) **EP 1 057 457 B1**
(45) Date de publication et mention de la délivrance du brevet: **14.07.2004**
(21) Numéro de dépôt: 00401525.1
(22) Date de dépôt: 30.05.2000
(51) Int. Cl.: A61F 2/04

(54) **Prothèse de dérivation biliaire**
Gallenshunt
Biliary shunt prosthesis

(30) Priorité: 03.06.1999 FR 9907006
(43) Date de publication de la demande: 06.12.2000
(73) Titulaire: Benelouezzane, Bourhane-Eddine, Constantine 25000 (DZ)
(72) Inventeur: Benelouezzane, Bourhane-Eddine, Constantine 25000 (DZ)
(74) Mandataire: Faber, Jean-Paul

(56) Documents cités:
- EP-A- 0 420 996
- US-A- 2 127 903
- US-A- 3 835 863
- US-A- 4 142 528
- US-A- 4 306 545
- US-A- 4 675 008
- US-A- 5 776 160

## Description

La présente invention a pour objet une prothèse de dérivation biliaire.

La présente invention se rapporte à une prothèse destinée à être utilisée pour des pathologies telles que les sténoses segmentaires des voies biliaires, les sténoses des anastomoses bilio-digestives, les tumeurs des voies biliaires, les tumeurs du carrefour bilio-pancréatique et les cholangites sclérosantes extra-hépatiques.

La technique antérieure connaît une prothèse (voir brevet européen n° 0 420 996 et brevet US n° 2 127 903) qui est constituée d'un élément tubulaire destiné à être inséré dans le canal cholédoque et à partir d'un point intermédiaire duquel s'étend un second élément tubulaire destiné à déboucher dans le duodénum ou le jéjunum.

De telles prothèses sont difficiles à fixer et souvent douloureuses pour le patient.

L'un des buts de l'invention est de remédier à ces inconvénients.

La prothèse, selon l'invention, est du type comportant un premier segment tubulaire destiné à être inséré dans le canal cholédoque et d'un second segment tubulaire s'étendant à partir d'un point intermédiaire du premier segment et destiné, par son extrémité, à être inséré dans le duodénum ou le jéjunum la partie dudit second segment voisine dudit premier segment tubulaire étant filetée, ladite prothèse étant caractérisée en ce qu'elle comporte une bague fixe percée de trous et disposée au voisinage de l'extrémité du second segment tubulaire adjacente au premier segment tubulaire, ladite partie filetée du second segment tubulaire comportant une bague taraudée percée de trous, tandis que la partie du second segment tubulaire voisine de l'extrémité destinée à être insérée dans le duodénum ou le jéjunum comporte un filetage sur lequel est montée une bague taraudée pourvue de trous, une bague fixe percée de trous étant prévue sur la partie du second segment tubulaire adjacente à l'extrémité destinée à être insérée dans le duodénum ou le jéjunum.

Grâce à cette disposition, on réalise une double voie de drainage biliaire ; en effet, le premier segment tubulaire constitue une première voie, tandis que le second segment tubulaire constitue une deuxième voie qui ramène la bile vers le segment du tube digestif le plus accessible; de plus on peut facilement amarrer la prothèse avec des fils chirurgicaux traversant les trous des bagues et les tissus adjacents.

Enfin, l'une des bagues fixes permet d'assurer l'ancrage de la partie de la prothèse insérée dans le duodénum ou le jéjunum et l'autre dans le canal cholédoque.

De plus, une telle prothèse permet un drainage interne et peut être maintenue dans le corps durant une longue période.

De préférence, le premier segment comporte une extrémité taillée en biseau destinée à être tournée vers les voies biliaires intra-hépatiques.

Suivant une caractéristique de détail, l'extrémité taillée en biseau comporte une cloison sagittale. Grâce à cette caractéristique, on évite l'obstruction de l'ouverture par des fragments tumoraux, des caillots ou des calculs.

Afin de faciliter la mise en place du premier segment tubulaire dans le canal cholédoque, la surface latérale du premier segment tubulaire est striée longitudinalement.

Suivant encore une autre caractéristique, l'extrémité du premier segment tubulaire, opposée à celle destinée à être tournée du côté des voies biliaires intra-hépatiques est taillée en biseau, le biseau étant décalé angulairement par rapport au biseau de l'extrémité destinée à être tournée du côté desdites voies biliaires intra-hépatiques. Ainsi, on évite d'obstruer le canal pancréatique.

De préférence, la surface latérale du premier segment tubulaire comporte des ouvertures qui peuvent avoir une forme triangulaire avec une pointe tournée vers l'extrémité du premier segment tubulaire destiné à être tourné du côté des voies biliaires intra-hépatiques, ces ouvertures étant destinées à constituer des collecteurs secondaires de la bile.

Suivant une variante de réalisation, l'extrémité du premier segment tubulaire, opposée à celle destinée à être tournée vers les voies biliaires intra-hépatiques, est coudée et est mobile angulairement. Une telle variante a pour avantage de pouvoir traiter les sténoses des anastomoses hépatico-jéjunales.

Suivant encore une autre caractéristique, le second segment tubulaire présente une partie intermédiaire en accordéon. Ainsi, ce second segment tubulaire peut avoir une certaine souplesse et une certaine mobilité dans le corps.

Suivant encore une autre caractéristique, l'extrémité du second segment tubulaire destinée à être insérée dans le duodénum ou le jéjunum est coudée et comporte des fentes latérales allongées.

Afin de pouvoir vérifier la bonne mise en place du premier segment tubulaire et éventuellement le lavage de la voie biliaire au cours de l'intervention et être assuré de sa perméabilité, l'extrémité du second segment tubulaire comporte un cathéter, celui-ci étant retiré avant l'insertion de l'extrémité du second segment tubulaire dans le duodénum ou le jéjunum.

Les faces des bagues taraudées, tournées en regard des bagues fixes correspondantes, peuvent être revêtues d'un tissu non résorbable afin de favoriser leur adhésivité aux parois correspondantes et éviter ainsi les fuites biliaires et digestives.

L'invention prévoit, également, un accessoire destiné à faciliter la pose de la prothèse telle que ci-dessus définie, ledit accessoire étant constitué d'un manche solidaire, à une extrémité, d'une tige flexible formant un coude avec ledit manche et dont l'extrémité libre comporte une série de protubérances.

Un tel accessoire introduit dans le canal cholédoque permet de dilater celui-ci et ainsi de mettre en place aisément le premier segment tubulaire dans ledit canal cholédoque.

De préférence, les protubérances sont réalisées en silicone, la tige étant gainée de silicone.

Enfin, les protubérances ont une section hexagonale.

L'invention va maintenant être décrite avec plus de détails en se référant à un mode de réalisation particulier donné à titre d'exemple seulement et représenté aux dessins annexés, dans lesquels :
Figure 1 est une vue en perspective de la prothèse.
Figure 2 est une vue à plus grande échelle d'une extrémité du premier segment tubulaire.
Figure 3 est une vue en coupe suivant la ligne 3-3 de la figure 2.
Figure 4 montre en perspective l'autre extrémité du premier segment tubulaire suivant une variante de réalisation.
Figure S est une vue en perspective de l'une des bagues.
Figure 6 montre à plus grande échelle un détail du second segment tubulaire.
Figure 7 montre à plus grande échelle en perspective, l'extrémité libre du second segment tubulaire.
Figure 8 est une vue en perspective d'un accessoire destiné à faciliter la pose de la prothèse.
Figure 9 est une vue en coupe à plus grande échelle suivant la ligne 9-9 de la figure 8.

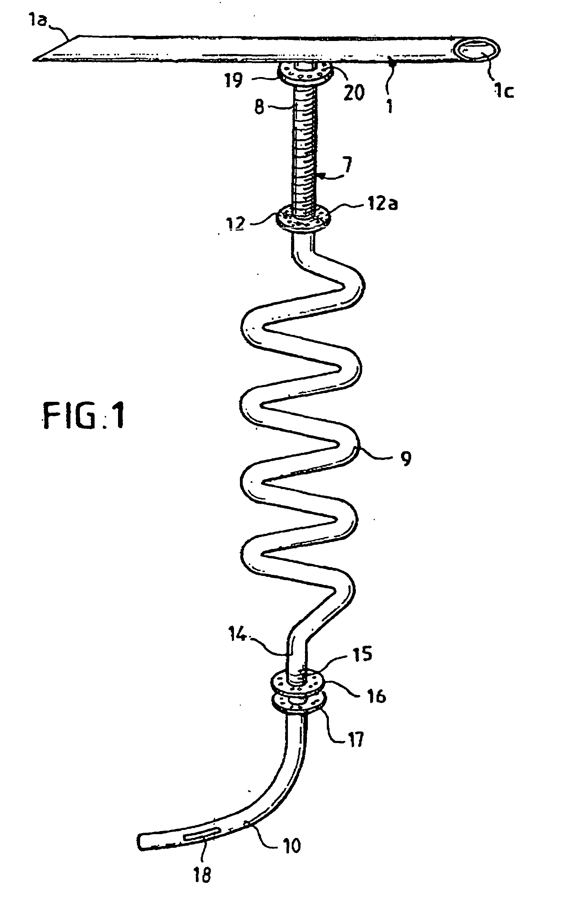

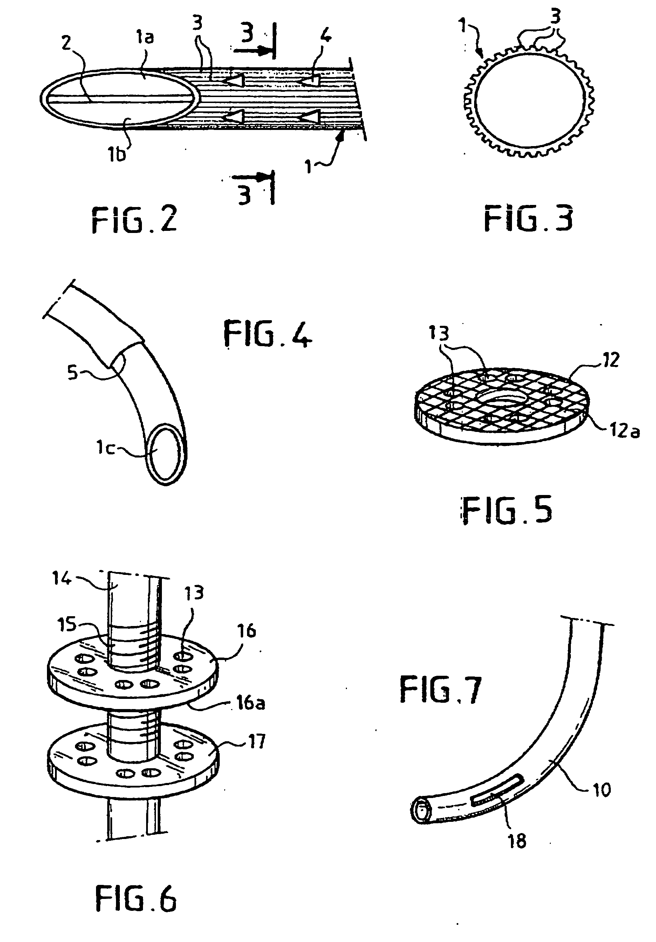

La prothèse représentée à la figure 1 comprend un premier segment tubulaire 1 dont une extrémité 1a (voir figure 2) est coupée en biseau, l'ouverture 1b du biseau présentant une cloison sagittale 2.

Le premier segment tubulaire 1 (voir figure 3) comporte des stries longitudinales 3 et des ouvertures 4 présentant une forme triangulaire dont un sommet est tourné vers l'ouverture en biseau 1b.

L'autre extrémité 1c du premier segment tubulaire 1 est coupée en biseau, celui-ci étant décalé angulairement de 90° par rapport au biseau 1b.

Suivant une variante de réalisation (voir figure 4), l'extrémité 1c peut décrire une courbe et être reliée mobile le long d'une liaison 5 de manière que la section terminale puisse avoir une certaine mobilité de l'ordre de 60°.

En un point intermédiaire de la longueur du premier segment tubulaire 1 s'étend un second segment tubulaire 7 qui s'ouvre dans le premier segment 1 et qui présente une section 8 filetée extérieurement et prolongée par une section 9 en accordéon finissant par une section terminale coudée 10.

A l'extrémité de la section filetée 8, adjacente au premier segment 1, est fixée une bague 19 percée de trous 20.

Sur la section filetée 8 est montée une bague taraudée 12 percée de trous 13 (voir figure 5) pour le passage de fils chirurgicaux, la face 12a de ladite bague 12, tournée en regard de la bague 19, étant revêtue d'un tissu tressé non résorbable, par exemple un tissu connu dans le commerce sous la dénomination "Dacron".

La section 9, de par sa forme, donne à la prothèse une certaine élasticité.

L'extrémité de la section 9, opposée à celle adjacente à la section filetée 8, présente une section rectiligne 14 avec un filetage 15 sur lequel se visse une bague taraudée 16 du même type que la bague 12 et dont la face 16a, tournée vers la section terminale 10, est revêtue d'un tissu tressé.

A l'extrémité de la section rectiligne 14 est fixée une bague 17 du même type que la bague 19.

La section terminale coudée 10 est percée d'une ouverture à son extrémité libre et comporte deux fentes latérales allongées 18 de drainage.

Dans la section terminale coudée 10 est logé un cathéter permettant d'injecter, dans la prothèse, un produit de contraste afin de vérifier, par des radiographies au cours de la pose de la prothèse, sa bonne mise en place.

Ce cathéter permet, également, le lavage de la voie biliaire au cours de l'opération afin de vérifier sa perméabilité et il est retiré avant la phase finale de l'opération.

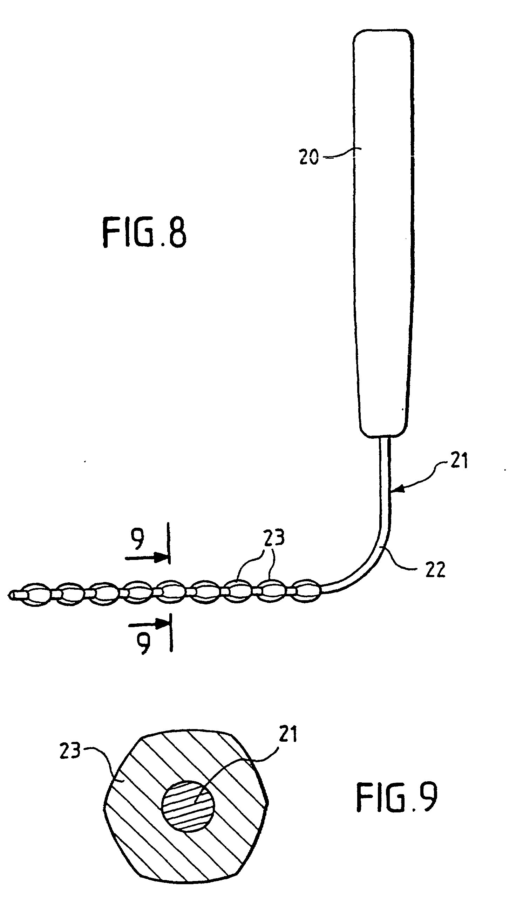

Les figures 8 et 9 montrent un accessoire utilisable avant la pose de la prothèse et destiné à faciliter la mise en place dans le canal cholédoque du premier segment 1.

Cet accessoire est constitué d'un manche 20 dans lequel est noyée une extrémité d'une tige 21 qui présente un coude 22, la tige 21 est gainée d'un silicone dans lequel sont formées des protubérances 23 s'étendant depuis le coude jusqu'à l'extrémité libre.

Les protubérances 23 ont, en section, une forme hexagonale et présentent un diamètre qui peut varier entre 3 et 8 mm.

La partie de la tige 21 comportant les protubérances peut avoir une longueur de l'ordre de 10 cm.

Dans la pratique, on pourra disposer d'une série d'accessoires avec des dimensions des protubérances différentes, le praticien sélectionnant l'accessoire correspondant à la section interne du canal cholédoque du patient.

Pour poser la prothèse, selon l'invention, on pratique une petite incision dans le canal cholédoque et on effectue une intubation trans-tumorale en engageant, par l'incision, l'extrémité de l'accessoire pourvu des protubérances 23, celles-ci, grâce à leur forme et à leur texture, n'étant pas traumatisantes.

Après retrait des protubérances, on insère toujours par l'incision le premier segment 1 de la prothèse afin que l'extrémité la soit tournée du côté des voies biliaires intra-hépatiques, tandis que l'extrémité 1c est tournée vers l'extrémité distale du cholédoque, le biseau de cette extrémité évitant toute obstruction des canaux pancréatiques.

On fixe ensuite le premier segment tubulaire 1 en amarrant la bague 19 à la face interne de la paroi digestive et, après vissage de la bague 12 pour l'amener contre les tissus avoisinants, on la fixe avec des fils chirurgicaux traversant les trous 13.

Par injection par le cathéter d'un produit de contraste, on peut vérifier par radiographie que le premier segment est convenablement mis en place, ledit cathéter étant ensuite retiré.

On procède ensuite à la mise en place dans le duodénum de la section 10 en pratiquant, dans celui-ci, une brèche permettant l'insertion de la section 10, la bague 17 étant insérée dans ladite brèche et une bourse étant formée autour de celle-ci. On visse ensuite la bague 16 pour l'amener au voisinage du tissu et on procède à l'amarrage en la fixant par des fils chirurgicaux traversant les trous de ladite bague 16.

Une telle prothèse, pour le traitement de tumeurs, peut être installée définitivement, par contre pour des affections bénignes, elle peut être retirée par voie chirurgicale.

La prothèse, selon l'invention, est réalisée en silicone, dont la tolérance est reconnue. Elle présente l'avantage, par rapport aux prothèses existantes, de réaliser une double voie de drainage, soit une première voie par le premier segment 1 et une seconde voie par le second segment 7 et qui amène la bile directement au duodénum.

Compte tenu des stries formées dans la surface latérale du premier segment tubulaire 1, le glissement dans le canal cholédoque est facilité.

La cloison 2 permet d'éviter une obstruction de l'ouverture 1b par des fragments tumoraux, des caillots ou des calculs.

Les ouvertures 4 constituent des collecteurs secondaires de la bile.

La prothèse, dont l'extrémité 1c du premier segment tubulaire 1 décrit une courbe (voir figure 4), peut permettre de traiter les sténoses des anastomoses hépatico-jujénales qui sont inaccessibles aux dilatations endoscopiques.

La prothèse, selon l'invention, peut également constituer un moyen thérapeutique d'urgence et permettre d'éviter, dans une certaine mesure, d'avoir recours aux anastomoses bilio-digestives, ce qui permet d'éviter les risques de fistules digestives.

La prothèse peut également être utilisée pour le traitement des fistules digestives, le premier segment 1 et la bague 19 étant introduits dans l'orifice fistuleux du tube digestif, estomac, intestin, duodénum, la bague 19 étant amarrée contre la face interne de la paroi digestive par des fils chirurgicaux.

On procède ensuite au vissage et à l'amarrage de la bague 12 qui est amenée contre la face externe du tube digestif et fixée par des fils chirurgicaux. Les lèvres de l'orifice fistuleux sont suturées, celui-ci se trouvant pris entre les deux bagues 19 et 12 et ainsi fermé.

Le drainage est assuré par les sections 7, 9 et 10 de sorte que la pression exercée sur l'orifice est allégée ce qui lui permet une cicatrisation progressive.

Après un délai d'environ trois mois, on peut procéder chirurgicalement à l'ablation de la prothèse, les orifices résiduels étant fermés par une simple suture, le tissu digestif étant, en principe, sain au bout de trois mois.

Les faces 12a et 16a, avec leur revêtement, favorisent l'adhésivité contre les parois et évitent les fuites biliaires et digestives.

Bien entendu, l'invention n'est pas limitée au mode de réalisation qui vient d'être décrit et représenté. On pourra y apporter de nombreuses modifications de détail sans sortir pour cela du cadre de l'invention, définie par les revendications suivantes.

## Revendications

1. Prothèse de dérivation biliaire du type comprenant un premier segment tubulaire (1) destiné à être inséré dans le canal cholédoque et d'un second segment tubulaire (7) s'étendant à partir d'un point intermédiaire du premier segment (1) et destiné, par son extrémité, à être inséré dans le duodénum ou le jéjunum la partie dudit second segment tubulaire, voisine dudit premier segment tubulaire (1), étant filetée, **caractérisée en ce qu'**elle comporte une bague fixe (19) percée de trous (20) disposée au voisinage de l'extrémité du second segment tubulaire (7), adjacente au premier segment tubulaire (1), ladite partie filetée du second segment tubulaire comportant une bague taraudée (12) percée de trous (13), tandis que la partie du second segment tubulaire (7), voisine de l'extrémité destinée à être insérée dans le duodénum ou le jéjunum comporte un filetage (15) sur lequel est montée une bague taraudée (16) pourvue de trous, une bague fixe (17) percée de trous étant prévue sur la partie du second segment tubulaire (7) adjacente à l'extrémité destinée à être insérée dans le duodénum ou le jujénum.

2. Prothèse de dérivation biliaire, selon la revendication 1, **caractérisée en ce que** le premier segment (1) comporte une extrémité taillée en biseau (1b) destinée à être tournée vers les voies biliaires intra-hépatiques.

3. Prothèse de dérivation biliaire, selon la revendication 2, **caractérisée en ce que** l'extrémité taillée en biseau (1b) comporte une cloison sagittale (2).

4. Prothèse de dérivation biliaire, selon la revendication 1, **caractérisée en ce que** la surface latérale du premier segment tubulaire (1) est striée longitudinalement.

5. Prothèse de dérivation biliaire, selon les revendications 1 et 2, **caractérisée en ce que** l'extrémité du premier segment tubulaire (1), opposée à celle destinée à être tournée du côté des voies biliaires intra-hépatiques, est taillée en biseau (1c), le biseau étant décalé angulairement par rapport au biseau de l'extrémité destinée à être tournée du côté desdites voies biliaires intra-hépatiques.

6. Prothèse de dérivation biliaire, selon la revendication 1, **caractérisée en ce que** la surface latérale du premier segment présente des ouvertures (4).

7. Prothèse de dérivation biliaire, selon les revendications 1 et 6, **caractérisée en ce que** les ouvertures (4) ont une forme triangulaire dont une pointe est tournée vers l'extrémité du premier segment tubulaire (1) destinée à être tournée du côté des voies biliaires intra-hépatiques.

8. Prothèse de dérivation biliaire, selon la revendication 1, **caractérisée en ce que** l'extrémité du premier segment tubulaire (1) opposée à celle destinée à être tournée vers les voies biliaires intra-hépatiques est coudée et est mobile angulairement.

9. Prothèse de dérivation biliaire, selon la revendication 1, **caractérisée en ce que** le second segment tubulaire (7) présente une partie intermédiaire en accordéon (9).

10. Prothèse de dérivation biliaire, selon la revendication 1, **caractérisée en ce que** l'extrémité du second segment tubulaire (7) destinée à être insérée dans le duodénum ou le jéjunum est coudée et comporte des fentes latérales allongées (18).

11. Prothèse de dérivation biliaire, selon la revendication 1, **caractérisée en ce que** l'extrémité du second segment tubulaire (7) comporte un cathéter.

12. Prothèse de dérivation biliaire, selon la revendication 1, **caractérisée en ce que** les faces des bagues taraudées (12a et 16a), tournées en regard des bagues fixes correspondantes (19 et 17), sont revêtues d'un tissu non résorbable.

13. Accessoire pour faciliter la pose de la prothèse de dérivation biliaire, selon la revendication 1 et/ou l'une quelconque des revendications 2 à 12, **caractérisé en ce que** ce qu'il est constitué d'un manche (20) solidaire, à une extrémité, d'une tige flexible (21) formant un coude avec ledit manche et dont l'extrémité libre comporte une série de protubérances (23).

14. Accessoire pour faciliter la pose de la prothèse de dérivation biliaire, selon la revendication 13, **caractérisé en ce que** les protubérances sont réalisées en silicone, la tige (21) étant gainée de silicone.

15. Accessoire pour faciliter la pose de la prothèse de dérivation biliaire, selon les revendications 13 et 14, **caractérisé en ce que** les protubérances ont une section hexagonale.

## Patentansprüche

1. Gallenshunt jener Art, die ein in den Hauptgallengang einzuführendes erstes röhrenförmiges Segment (1) und ein sich von einer Zwischenstelle des ersten Segments (1) aus erstreckendes, mit seinem Ende in den Zwölffingerdarm oder den Leerdarm einzuführendes zweites röhrenförmiges Segment (7) umfasst, wobei der neben dem ersten röhrenförmigen Segment (1) liegende Teil des zweiten röhrenförmigen Segments mit einem Gewinde versehen ist, **dadurch gekennzeichnet, dass** er einen mit Löchern (20) versehenen feststehenden Ring (19) aufweist, der in der Nähe des Endes des zweiten röhrenförmigen Segments (7) neben dem ersten röhrenförmigen Segment (7) angeordnet ist, wobei der Gewindeteil des zweiten röhrenförmigen Segments einen mit Innengewinde versehenen Ring (12) aufweist, der mit Löchern (13) versehen ist, während der Teil des zweiten röhrenförmigen Segments (7) neben dem in den Zwölffingerdarm oder den Leerdarm einzuführenden Ende ein Gewinde (15) aufweist, an dem ein mit Innengewinde versehener, Löcher aufweisender Ring (16) angebracht ist, wobei ein mit Löchern versehener feststehender Ring (17) an dem neben dem in den Zwölffingerdarm oder den Leerdarm einzuführenden Ende liegenden Teil des zweiten röhrenförmigen Segments (7) vorgesehen ist.

2. Gallenshunt nach Anspruch 1, **dadurch gekennzeichnet, dass** das erste Segment (1) ein abgeschrägtes Ende (1b) aufweist, das zu den intrahepathischen Gallenwegen weisen soll.

3. Gallenshunt nach Anspruch 2, **dadurch gekennzeichnet, dass** das abgeschrägte Ende (1b) eine Sagittaltrennwand (2) aufweist.

4. Gallenshunt nach Anspruch 1, **dadurch gekennzeichnet, dass** die Seitenfläche des ersten röhrenförmigen Segments (1) in Längsrichtung gerillt ist.

5. Gallenshunt nach den Ansprüchen 1 und 2, **dadurch gekennzeichnet, dass** das Ende des ersten röhrenförmigen Segments (1), das dem, das zu den intrahepathischen Gallengängen weisen soll, gegenüberliegt, abgeschrägt ist (1c), wobei die Abschrägung winkelförmig zur Abschrägung des Endes versetzt ist, das zu den intrahepathischen Gallengängen weisen soll.

6. Gallenshunt nach Anspruch 1, **dadurch gekennzeichnet, dass** die Seitenfläche des ersten Segments Öffnungen (4) aufweist.

7. Gallenshunt nach den Ansprüchen 1 und 6, **dadurch gekennzeichnet, dass** die Öffnungen (4) die Form eines Dreiecks aufweisen, von dem eine Spitze zu dem Ende des ersten röhrenförmigen Segments (1) weist, das zu den intrahepathischen Gallengängen weisen soll.

8. Gallenshunt nach Anspruch 1, **dadurch gekennzeichnet, dass** das Ende des ersten röhrenförmigen Segments (1), das dem gegenüberliegt, das zu den intrahepathischen Gallengängen weisen soll, gebogen und winkelförmig beweglich ist.

9. Gallenshunt nach Anspruch 1, **dadurch gekennzeichnet, dass** das zweite röhrenförmige Segment (7) einen ziehharmonikaförmigen Zwischenteil (9) aufweist.

10. Gallenshunt nach Anspruch 1, **dadurch gekennzeichnet, dass** das in den Zwölffingerdarm oder den Leerdarm einzuführende Ende des zweiten röhrenförmigen Segments (7) gebogen ist und längliche Seitenschlitze (18) aufweist.

11. Gallenshunt nach Anspruch 1, **dadurch gekennzeichnet, dass** das Ende des zweiten röhrenförmigen Segments (7) einen Katheter aufweist.

12. Gallenshunt nach Anspruch 1, **dadurch gekennzeichnet, dass** die Flächen (12a und 16a) der mit Innengewinde versehenen Ringe, die zu den entsprechenden feststehenden Ringen (19 und 17) hin weisen, mit einem nicht resorbierbaren Gewebe überzogen sind.

13. Zubehörteil zur Erleichterung der Anordnung des Gallenshunts nach Anspruch 1 und/oder einem der Ansprüche 2 bis 12, **dadurch gekennzeichnet, dass** es aus einem Halter (20) besteht, der mit einem Ende fest mit einer flexiblen Stange (21) verbunden ist, die mit dem Halter ein Winkelstück bildet und deren freies Ende eine Reihe von Vorsprüngen (23) aufweist.

14. Zubehörteil zur Erleichterung der Anordnung des Gallenshunts nach Anspruch 13, **dadurch gekennzeichnet, dass** die Vorsprünge aus Silikon hergestellt sind, wobei die Stange (21) mit Silikon ummantelt ist.

15. Zubehörteil zur Erleichterung der Anordnung des Gallenshunts nach den Ansprüchen 13 und 14, **dadurch gekennzeichnet, dass** die Vorsprünge einen sechseckigen Querschnitt aufweisen.

## Claims

1. Biliary shunt prosthesis of the type comprising a first tubular segment (1) designed to be inserted in the bile duct and a second tubular segment (7) extending from an intermediate point on the first segment (1) and designed to have its end inserted in the duodenum or the jejunum, the section of said second tubular segment, next to said first tubular segment (1), being threaded, **characterised in that** it comprises a fixed ring (19) pierced with holes (20) arranged near the end of the second tubular segment (7), adjacent to the first tubular segment (1), comprising a tapped ring (12) pierced with holes (13), while the section of the second tubular segment (7), next to the end designed to be inserted in the duodenum or the jejunum comprises a thread (15) on which a tapped ring (16) provided with holes is mounted, a fixed ring (17) pierced with holes being provided on the section of the second tubular segment (7) adjacent to the end designed to be inserted in the duodenum or jejunum.

2. Biliary shunt prosthesis according to claim 1, **characterised in that** the first segment (1) comprises a bevelled end (1b) designed to be turned towards the intra-hepatic biliary ducts.

3. Biliary shunt prosthesis according to claim 2, **characterised in that** the bevelled end (1b) comprises a sagittal partition (2).

4. Biliary shunt prosthesis according to claim 1, **characterised in that** the lateral surface of the first tubular segment (1) is grooved longitudinally.

5. Biliary shunt prosthesis according to claims 1 and 2, **characterised in that** the end of the first tubular segment (1), opposite the one designed to be turned towards the intra-hepatic biliary ducts, is bevelled (1c), the bevel being offset at an angle in relation to the bevel of the end designed to be turned towards said intra-hepatic biliary ducts.

6. Biliary shunt prosthesis according to claim 1, **characterised in that** the lateral surface of the first segment has openings (4).

7. Biliary shunt prosthesis according to claims 1 and 6, **characterised in that** the openings (4) are triangular in form with one point turned towards the end of the first tubular segment (1) designed to be turned towards the intra-hepatic biliary ducts.

8. Biliary shunt prosthesis according to claim 1, **characterised in that** the end of the first tubular segment (1) opposite the one designed to be turned towards the intra-hepatic biliary ducts is bent and moveable at an angle.

9. Biliary shunt prosthesis according to claim 1, **characterised in that** the second tubular segment (7) has an intermediate corrugated section (9).

10. Biliary shunt prosthesis according to claim 1, **characterised in that** the end of the second tubular segment (7) designed to be inserted in the duodenum or jejunum is bent and comprises elongated lateral slits (18).

11. Biliary shunt prosthesis according to claim 1, **characterised in that** the end of the second tubular segment (7) comprises a catheter.

12. Biliary shunt prosthesis according to claim 1, **characterised in that** the faces of the tapped rings (12a and 16a), turned towards the corresponding fixed rings (19 and 17), are covered with a non-resorbent fabric.

13. Accessory to facilitate fitting of the biliary shunt prosthesis according to claim 1 and/or any one of claims 2 to 12, **characterised in that** it consists of an integral sleeve (20) at one end of a flexible rod (21) forming an elbow with said sleeve of which the free end comprises a series of protuberances (23).

14. Accessory to facilitate fitting of the biliary shunt duct according to claim 13, **characterised in that** the protuberances are made of silicone, the rod (21) being sheathed in silicone.

15. Accessory to facilitate fitting of the biliary shunt prosthesis according to claims 13 and 14, **characterised in that** the protuberances are hexagonal in cross-section.
